# EUROPÄISCHE PATENTSCHRIFT

(11) **EP 2 303 355 B1**
(45) Veröffentlichungstag und Bekanntmachung des Hinweises auf die Patenterteilung: **04.06.2014**
(21) Anmeldenummer: 09772146.8
(22) Anmeldetag: 30.06.2009
(51) Int. Cl.: A61M 1/28

(54) **VORRICHTUNG ZUR PERITONEALDIALYSE**
DEVICE FOR PERITONEAL DIALYSIS
SYSTÈME POUR DIALYSES PÉRITONÉALES

(30) Priorität: 04.07.2008 DE 102008031662
(43) Veröffentlichungstag der Anmeldung: 06.04.2011
(73) Patentinhaber: Fresenius Medical Care Deutschland GmbH, 61352 Bad Homburg (DE)
(72) Erfinder: HEDMANN, Frank, 97332 Volkach (DE); KLATTE, Stephan, 31582 Nienburg (Weser) (DE)
(74) Vertreter: Laufhütte, Dieter
(86) Internationale Anmeldenummer: PCT/EP2009/004721
(87) Internationale Veröffentlichungsnummer: WO 2010/000446

(56) Entgegenhaltungen:
- WO-A1-01/58509
- WO-A1-99/06082
- WO-A1-2005/035023
- US-A- 5 004 459
- US-A1- 2004 019 312
- US-A1- 2007 106 247

## Beschreibung

Die Erfindung betrifft eine Vorrichtung zur Peritonealdialyse mit einer Einrichtung zur regelmäßigen Abgabe und Wiederaufnahme von Dialysat. Des weiteren betrifft die Erfindung ein Meßdisposable und ein Meßverfahren zur Messung des intraperitonealen Drucks.

Die Peritonealdialyse ist eine Variante der künstlichen Blutwäsche. Hier wird das gut durchblutete Bauchfell (Peritonium) des Patienten als körpereigene Filtermembran verwendet. Über einen Katheter wird Dialysat in die Bauchhöhle eingeführt. Nach dem Prinzip der Osmose werden Harnbestandteile dem Blut entzogen und gelangen in die Bauchhöhle. Nach einigen Stunden wird das Dialysat mit den Harnbestandteilen wieder aus der Bauchhöhle ausgelassen.

Zur Durchführung der Peritonealdialyse gibt es grundsätzlich verschiedene Möglichkeiten. Bei der kontinuierlichen ambulanten Peritonealdialyse (CAPD) wechseln die Patienten selbst etwa vier bis fünfmal am Tag das Dialysat. Bei der automatischen Peritonealdialyse (APD) übernimmt eine Vorrichtung, der sogenannte Cycler, den automatischen Beutelwechsel über Nacht, so dass der Patient tagsüber noch unabhängig ist.

Bei der automatischen Peritonealdialyse, bei der die Bauchhöhle mit Hilfe des vorgenannten Cyclers befüllt wird, wird üblicherweise über eine Volumensteuerung darauf geachtet, dass dem Patienten nur ein maximales Füllvolumen verabreicht wird. Dieses maximale Füllvolumen beträgt bei einem durchschnittlichen Erwachsenen beispielsweise 3.500 ml. Üblicherweise werden standardisierte Werte für das Füllvolumen verwendet, da der Aufwand für eine experimentelle Bestimmung des patientenindividuellen Füllvolumens vermieden werden soll.

Für die Ermittlung eines Volumens kann beispielesweise auch eine Druckmessung, insbesondere eine Messung des intraperitonealen Drucks herangezogen werden. So beschreibt die WO 01/58509 ein APD-System und ein Verfahren zum Überwachen und Steuern von Peritonealdialysebehandlungen. Das System sieht hierzu zwischen dem Dialysat-Container und dem Patienten einen ersten Sensor und zwischen Drain-Container und dem Patienten einen zweiten Sensor vor, zur Bestimmung des intraperitonealen Drucks während der Füll-, Dwell- und Drainphase der Behandlung.

In der WO 99/06082 ist ebenfalls eine APD-Vorrichtung offenbart zur Überwachung des intraperitonealen Drucks. Hierfür verfügt die Vorrichtung über einen sogenannten "in-line pressure transducter" zur Druckmessung.

Auch die US 2007/0106247 betrifft die Ermittlung des intraperitonealen Drucks wobei die hydrostatische Druckhöhe, anhand eines verstellbaren Stabs, variiert und mittels eines "in-line Drucksensors", aufgenommen werden kann.

In der WO 2005/035023 ist die kontinuierliche Bestimmung eines intraperitonealen Drucks während der Drainphase offenbart, wobei die Drainphase abgebrochen wird, sobald eine Variable einen Unterbrechungspunkt erreicht hat, an dem der Wert der Variablen sich radikal ändert.

Die US 5,004,459 beschreibt ein APD Gerät welches Mittel zur Bestimmung des Drucks und des Volumens im Peritoneum eines Patienten aufweist, wobei das Gerät weiterhin über ein Leitungssystem verfügt welches einen Entlüftungsfilter beinhaltet.

Aufgabe der vorliegenden Erfindung ist es nun, eine Vorrichtung zur Peritonealdialyse derart weiterzubilden, dass von der Vorrichtung automatisch das Volumen des jeweils abzugebenden Dialysats bestimmbar ist. Weiterhin soll ein entsprechendes Meßverfahren an die Hand gegeben werden.

Erfindungsgemäß wird diese Aufgabe dadurch gelöst, dass bei einer gattungsgemäßen Vorrichtung zur Peritonealdialyse eine Druckmeßeinrichtung zur Messung des intraperitonealen Drucks vorgesehen wird, wie dies Gegenstand von Anspruch 1 ist.

Unter dem intraperitonealen Druck versteht man den Innendruck des Peritoneums, der sich dadurch ergibt, dass die Bauchhöhle mit Dialysat gefüllt wird und auf das Peritoneum einen Gegendruck ausbildet, der wiederum dazu genutzt werden kann, den idealen Füllgrad für den individuellen Patienten zu bestimmen. Das Vorsehen der Druckmeßeinrichtung erlaubt eine druckgesteuerte Befüllung der Bauchhöhle unter Ausnutzung des individuell vorhandenen Volumens.

Bevorzugte Ausgestaltungen der Erfindung ergeben sich aus den sich an den Hauptanspruch anschließenden Unteransprüchen.

Demnach kann über die Druckmeßeinrichtung der statische und/oder dynamische Druck während des Befüllens meßbar sein.

Erfindungsgemäß weist die Druckmeßeinrichtung neben den Druckmeßsensoren eine Doppellumenleitung oder zwei parallele Leitungen auf. Von diesen ist eine der beiden Leitungen zur Umgebung über einen Filter derart abgeschlossen, dass der Umgebungsdruck zu dem vorrichtungsseitigen Drucksensor übertragbar ist.

Besonders vorteilhaft ist die Doppellumenleitung als Disposable ausgebildet, so dass sie nach jeder Behandlung ausgetauscht werden kann.

Die Vorrichtung enthält gemäß einer bevorzugten Ausgestaltung der Erfindung eine Steuereinheit, mittels der anhand der ermittelten Druckwerte die maschinelle Abgabe des Dialysats steuerbar ist.

Dabei kann auf der Steuereinheit bei Durchführung einer dynamischen Druckmessung eine Steuerung derart ablaufen, dass der Druck der Patientenleitung überwacht wird, wobei dieser gemessene Druck um die dynamischen Eigenschaften kompensiert und um den Druck der zweiten, den Umgebungsdruck messenden Leitung, vermindert wird. Bei dieser Messung sind die Flußwiderstände der Leitung und des Katheters bekannt, so dass diese Werte bei der Flußratenkompensation als dynamische Eigenschaften kompensiert werden können.

Alternativ kann hier in Form einer statischen Messung bei unterschiedlichen Füllständen der Druck der Patientenleitung überwacht werden. Hier wird also der Druck der Patientenleitung mittels der Druckmeßeinrichtung zyklisch überwacht, wobei dieser gemessene Druck um den Druck der zweiten Leitung, die den Umgebungsdruck mißt, vermindert wird.

Besonders vorteilhaft ist es bei Verwendung der Vorrichtung, dass mit dieser verschiedene Fehlerquellen ausgeschlossen werden können. Wird beispielsweise ein unerwarteter intraperitonealer Druck oder Druckanstieg verzeichnet, kann dies auf unterschiedliche Fehlerquellen hindeuten. So kann beispielsweise die Bauchhöhle des Patienten vor dem Befüllvorgang nicht vollständig entleert worden sein. Des weiteren könnte auch fälschlich ein Behandlungsprogramm für einen Erwachsenen ausgewählt worden sein, bei dem ein hohes Füllvolumen verabreicht werden muss, obwohl tatsächlich ein Kind an die Maschine angeschlossen ist.

Diese Fehler werden bei Nutzung der erfindungsgemäßen Vorrichtung sicher verhindert.

Ein Meßdisposable für die erfindungsgemäße Vorrichtung ist Gegenstand des Anspruchs 7 und des auf diesen zurückbezogenen Anspruchs 8.

Weitere Merkmale, Einzelheiten und Vorteile der Erfindung werden anhand eines in der Zeichnung dargestellten Ausführungsbeispiels näher erläutert. Es zeigen:
- Fig. 1:: eine Ausführungsform der erfindungsgemäßen Vorrichtung in rein schematischer Darstellung und
- Fig. 2:: ein Detail der Vorrichtung nach Fig. 1.

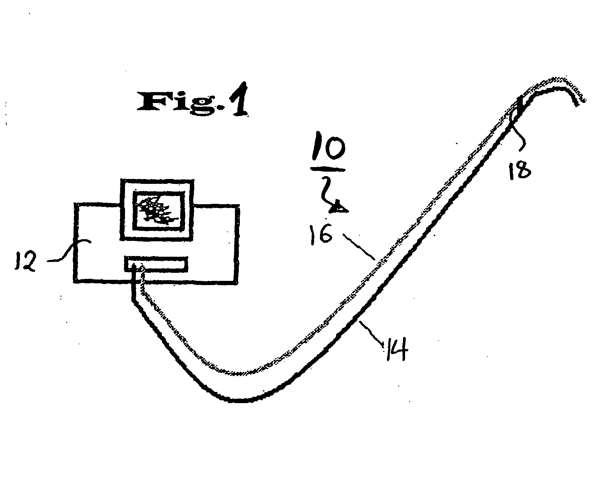

Die Vorrichtung 10 zur Peritonealdialyse entsprechend der hier in Fig. 1 vorgestellten Ausführungsvariante weist einen in seinem Aufbau an sich bekannten sogenannten Cycler 12 auf, der die Bauchhöhle des Patienten automatisch befüllt und entleert. An dem Cycler 12 schließt eine Patientenleitung 14 an. Neben der Patientenleitung 14, über die das Dialysat gefördert wird, ist eine zweite Leitung 16 als Meßleitung ausgeführt. Auf Seiten des Cyclers 12 sind die Enden der Leitungen 14 und 16 jeweils mit einem hier nicht näher dargestellten Drucksensor gekoppelt, so dass der jeweils in der Leitung herrschende Druck meßbar ist.

Voraussetzung für die Messung des intraperitonealen Druckes, das heißt des Innendruckes des Peritoneums, ist es, dass die zweite Leitung 16 als Meßleitung vollständig mit Dialysat gefüllt ist und auf gleicher Höhe wie das Ende der Patientenleitung endet, wobei die Meßleitung nur über eine hier nicht näher dargestellte Filtereinrichtung an ihrem freien Ende mit der Umgebung in Verbindung steht, so dass hier an dem freien Ende der Leitung 16 Umgebungsdruck anliegt.

Um sicherzustellen, dass das freie Ende der zweiten Leitung 16 in gleicher Höhe wie die Patientenleitung 14 ist, wird eine mechanische Verbindung 18 nahe dem Ende der Leitungen 14 und 16 vorgesehen. Diese kann beispielsweise aus einem Klipp bestehen.

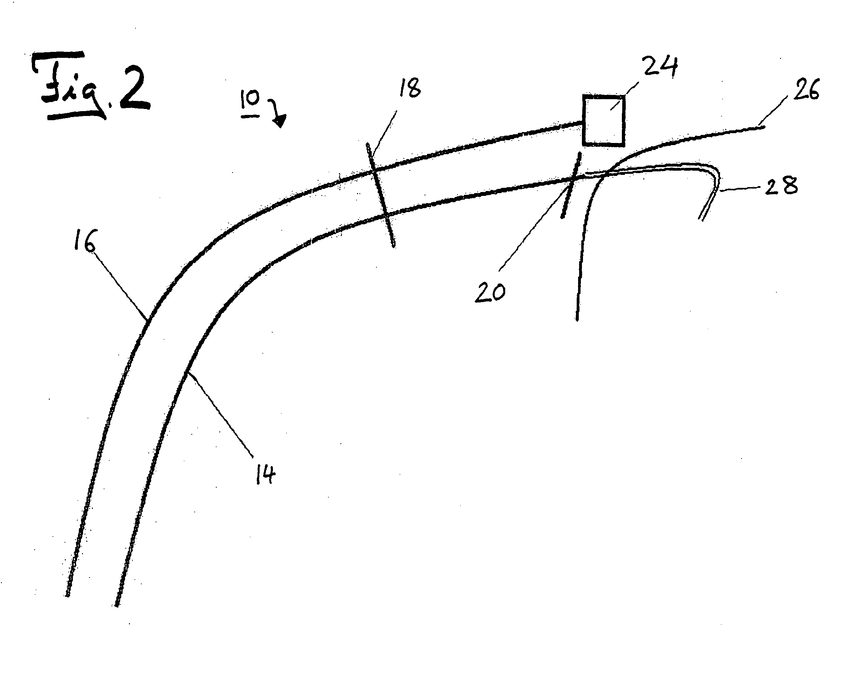

Wie in der Fig. 2 dargestellt endet die Leitung 14 mit ihrem freien Ende in einem Filter 24, das flüssigkeitsdicht und luftdurchlässig ist. Die Leitung 14 ist über einen Konnektor 20 mit einem die Bauchdecke 26 des Patienten durchdringenden Katheter 28 mit dem Peritoneum verbunden.

In hier nicht näher dargestellter Art und Weise weist der Cycler 12 eine Steuereinheit auf, mittels der anhand der ermittelten Druckwerte die maschinelle Abgabe des Dialysats steuerbar ist. In der Steuereinheit läuft die Steuerung bei zyklischer Messung derart, dass zunächst über den an der zweiten Leitung 16 angeordneten Drucksensor zunächst nur der reine statische Druck gemessen wird.

An dem anderen Drucksensor, der mit der Patientenleitung 14 verbunden ist, wird der intraperitoneale Druck zusätzlich zu dem statischen Druck gemessen. Somit lässt sich der intraperitoneale Druck bei dieser statischen Messung dadurch berechnen, dass der mit der zweiten Leitung 16 gemessene statische Druck abgezogen wird.

Bei einer Messung im Betrieb, das heißt einer dynamischen Messung, müssen zusätzlich die sogenannten dynamischen Größen in die Berechnung einbezogen werden. Diese ergeben sich bei der Durchströmung der Leitung und des Katheters durch die hier auftretenden Flußwiderstände. Die sogenannte Flußratenkompensation lässt sich für das vorhandene System ermitteln und damit rechnerisch in der Steuereinheit bei einer dynamischen Messung des Drucks berücksichtigen.

Entsprechend der Druckwerte wird die Bauchhöhle des Patienten mit der erfindungsgemäßen Vorrichtung druckgesteuert befüllt.

## Patentansprüche

1. Vorrichtung (10) zur Peritonealdialyse mit einer Einrichtung zur regelmäßigen Abgabe und Wiederaufnahme von Dialysat, wobei sie eine Druckmeseinrichtung zur Messung des intraperitonealen Drucks aufweist und wobei die Druckmesseinrichtung eine Doppellumenleitung oder zwei parallele Leitungen (14, 16) sowie Drucksensoren aufweist,
**dadurch gekennzeichnet, dass** beide Leitungen (14, 16) jeweils mit einem Drucksensor verbunden sind und eine der beiden Leitungen (14) zur Umgebung über einen Filter (24) so abgeschlossen ist, dass der Umgebungsdruck zu dem vorrichtungsseitigen Drucksensor übertragbar ist und der Filter (24) am freien Ende der einen Leitung (14) auf gleicher Höhe wie das Ende der anderen Leitung (16) vorgesehen ist.

2. Vorrichtung (10) nach Anspruch 1, **dadurch gekennzeichnet, dass** über die Druckmesseinrichtung der statische und/Oder dynamische Druck während des Befüllens messbar ist.

3. Vorrichtung (10) nach Anspruch 1 oder 2, **dadurch gekennzeichnet, dass** die Doppellumenleitung als Disposable ausgebildet ist.

4. Vorrichtung (10) nach einem der vorangegangenen Ansprüche, **dadurch gekennzeichnet, dass** sie eine Steuereinheit aufweist, mittels der anhand der ermittelten Druckwerte die maschinelle Abgabe des Dialysats steuerbar ist.

5. Vorrichtung (10) nach Anspruch 4, **dadurch gekennzeichnet, dass** auf der Steuereinheit eine Steuerung abläuft, bei der bei kontinuierlicher Druckmessung der Druck der Patientenleitung (14) überwacht wird, wobei dieser gemessene Druck um die dynamischen Eigenschsften kompensiert und um den Druck in der zweiten Leitung (16) vermindert wird.

6. Vorrichtung (10) nach Anspruch 4, **dadurch gekennzeichnet, dass** auf der Steuereinheit eine Steuerung abläuft, bei der bei statischer Druckmessung der Druck der Patientenleitung (14) zyklisch überwacht wird, wobei dieser gemessene Druck um den Druck der zweiten Leitung (16) vermindert wird.

7. Messdisposable für eine Vorrichtung (10) nach einem der Ansprüche 1 bis 6, **dadurch gekennzeichnet, dass** es zwei zumindest teilweise miteinander verbundene Leitungen (14, 16) oder eine Doppellumenleitung umfasst, von denen eine in einen Filter (24) mündet, während die andere beispielsweise über einen Konnektor (20) mit einem Katheter (28) verbindbar ist und die beiden Leitungen (14, 16) kurz vor ihrem freien Ende über eine mechanische Verbindung (18) miteinander verbunden sind.

8. Messdisposable nach Anspruch 7, **dadurch gekennzeichnet, dass** das Filter (24) flüssigkeitsdicht und luftdurchlässig ist.

## Claims

1. An apparatus (10) for peritoneal dialysis having a device for the regular dispensing and reuptake of dialysate, wherein it has a pressure measuring device for the measurement of the intraperitoneal pressure and wherein the pressure measuring device has a double lumen line or two parallel lines (14, 16) as well as pressure sensors,
**characterized in that** both lines (14, 16) are each connected to a pressure sensor and one of the two lines (14) is closed toward the environment via a filter (24) such that the ambient pressure can be transferred to the pressure sensor at the apparatus side and the filter (24) is provided at the free end of the one line (14) at the same level as the end of the other line (16).

2. An apparatus (10) in accordance with claim 1, **characterized in that** the static pressure and/or the dynamic pressure can be measured via the pressure measuring device during the filling.

3. An apparatus (10) in accordance with claim 1 or 2, **characterized in that** the double lumen line is made as a disposable.

4. An apparatus (10) in accordance with one of the preceding claims, **characterized in that** it has a control unit by means of which the dispensing by the machine of the dialysate is controllable based on the determined pressure values.

5. An apparatus (10) in accordance with claim 4, **characterized in that** a control runs on the control unit in which the pressure of the patient line (14) is monitored in a continuous pressure measurement, with this measured pressure being compensated by the dynamic properties and being reduced by the pressure in the second line (16).

6. An apparatus (10) in accordance with claim 4, **characterized in that** a control runs on the control unit in which the pressure of the patient line (14) is monitored cyclically in a static pressure measurement, with this measured pressure being reduced by the pressure in the second line (16).

7. A measurement disposable for an apparatus (10) in accordance with one of the claims 1 to 6, **characterized in that** it includes two lines (14, 16) which are at least partly connected to one another or a double lumen line and of which one opens into a filter (24), whereas the other can be connected to a catheter (28), for example via a connector (20) and both lines (14, 16) are connected to one another via a mechanical connection (18) just before their free ends.

8. A measurement disposable in accordance with claim 7 **characterized in that** the filter (24) is liquid-tight and air-permeable.

## Revendications

1. Système (10) pour dialyses péritonéales comprenant un dispositif destiné à distribuer et reprendre régulièrement du dialysat, le dispositif comportant un dispositif de mesure de la pression destiné à mesurer la pression intrapéritonéale et le dispositif de mesure de la pression comportant un conduit à double lumière ou deux conduits parallèles (14, 16) ainsi que des capteurs de pression,
**caractérisé en ce que** les deux conduits (14, 16) sont reliés respectivement à un capteur de pression et l'un des deux conduits (14) est séparé du milieu ambiant par le biais d'un filtre (24) de telle sorte que la pression ambiante peut être transmise au capteur de pression côté système, et le filtre (24) est prévu à l'extrémité libre de l'un des conduits (14) à la même hauteur que l'extrémité de l'autre conduit (16).

2. Système (10) selon la revendication 1, **caractérisé en ce que** la pression statique et/ou dynamique peut être mesurée par le dispositif de mesure de la pression pendant le remplissage.

3. Système (10) selon la revendication 1 ou 2, **caractérisé en ce que** le conduit à double lumière est conçu sous forme d'objet jetable.

4. Système (10) selon l'une des revendications précédentes, **caractérisé en ce qu'**il comporte une unité de commande au moyen de laquelle la distribution mécanique du dialysat peut être commandée à l'aide des valeurs de pression déterminées.

5. Système (10) selon la revendication 4, **caractérisé en ce qu'**une commande est exécutée sur l'unité de commande, selon laquelle la pression du conduit du patient (14) est surveillée par mesure continue de la pression, cette pression mesurée étant compensée par les propriétés dynamiques et réduite de la pression dans le second conduit (16).

6. Système (10) selon la revendication 4, **caractérisé en ce qu'**une commande est exécutée sur l'unité de commande, selon laquelle la pression du conduit du patient (14) est surveillée de manière cyclique par mesure statique de la pression, cette pression mesurée étant réduite de la pression dans le second conduit (16).

7. Objet de mesure jetable pour un système (10) selon l'une des revendications 1 à 6, **caractérisé en ce qu'**il comprend deux conduits (14, 16) reliés au moins en partie l'un à l'autre ou un conduit à double lumière, dont un débouche dans un filtre (24) tandis que l'autre peut être relié par exemple à un cathéter (28) par un connecteur (20) et les deux conduits (14, 16) sont reliés l'un à l'autre juste avant leur extrémité libre par le biais d'une liaison mécanique (18).

8. Objet de mesure jetable selon la revendication 7, **caractérisée en ce que** le filtre (24) est étanche au liquide et perméable à l'air.
